Europäisches Patentamt

(19) European Patent Office

Office européen des brevets

(11) Publication number: **0 235 460**
**A1**

(12) # EUROPEAN PATENT APPLICATION

(21) Application number: 86310002.0

(51) Int. Cl.⁴: **A 61 F 9/08**, G 09 B 21/00

(22) Date of filing: **22.12.86**

(30) Priority: **05.02.86 GB 8602761**

(71) Applicant: **Al-Basri, Akeel, 116 Willows Lane, Bolton Greater Manchester BL3 4AB (GB)**

(43) Date of publication of application: **09.09.87 Bulletin 87/37**

(84) Designated Contracting States: **DE FR GB IT**

(72) Inventor: **Al-Basri, Akeel, 116 Willows Lane, Bolton Greater Manchester BL3 4AB (GB)**

(54) **A blind person transformer of vision to sound.**

(57) This invention is a portable electronic instrument (1) providing the blind person with 2 sensor devices:

1) A colour sensor
2) An illumination sensor

Both sensors are contained in the sensor cylinder (2).

Operation of the colour sensor is controlled by depressing the first button (4) on the sensor cylinder (2) and placing the sensor surface (6) on the object whose colour is required.

Operation of the illumination sensor is controlled by depressing the second button (5) on the sensor cylinder (2). The sensor surface (6) will sense the level of illumination in its immediate surroundings.

The information sensed in either sensor, within the sensor cylinder (2) is passed via the attached cable (3) to the electronic hardware (1), where the information is analysed. As a result, the information originally sensed, for example, either a colour or a level of illumination, will be produced as electronically generated speech e.g. "BLUE" or "DARK" etc, through the ear-piece (7) or through the speaker (8), according to which sensor is used.

# A BLIND PERSONS TRANSFORMER OF VISION TO SOUND

This invention relates to apparatus and methods for transmitting information to the blind person by way of speech. The information transmitted will be concerning all colours of objects and levels of illumination.

The idea of the invention is to produce an electronic instrument providing the blind person with 2 sensor devices:-

      a)  A colour sensor

      b)  An illumination sensor

These sensors will inform the blind person through an ear-piece or through a speaker, by electronically generated speech, e.g.

1) When the colour sensor is in operation and is placed on an object, the user will hear the name of that colour e.g. "RED" or "BLUE" etc..

2) When the illumination sensor is in operation, then according to the level of illumination in the immediate surroundings, the user will hear words such as "DARK", "LIGHT" or "BRIGHT" etc..

The device will be fully portable, battery operated and simple to operate as required by the blind.

This instrument consists of a portable case (1) which contains all the electronic hardware. The two sensors, the colour sensor and the illumination sensor are contained in the sensor cylinder (2).

Operation of the colour sensor is controlled by depressing the first button (4) on the sensor cylinder (2) and placing the sensor surface (6) on the object whose colour is required.

Operation of the illumination sensor is controlled by depressing the second button (5) on the sensor cylinder (2). The sensor surface (6) will sense the level of illumination in its immediate surroundings.

The colour or illumination sensed from either sensor, within the sensor cylinder (2), is then passed as analogue to the electronic hardware (1) via the attached cable (3). The electronic hardware (1) will then convert the analogue information to digital information which is then analysed.

After this analysis, the information originally sensed, for example, either a colour or a level of illumination, will be produced as electronically generated speech, that is, "RED" , "BLUE" , "LIGHT" or "DARK" etc., through the ear-piece (7) or through the speaker (8) on the case of the instrument (1).

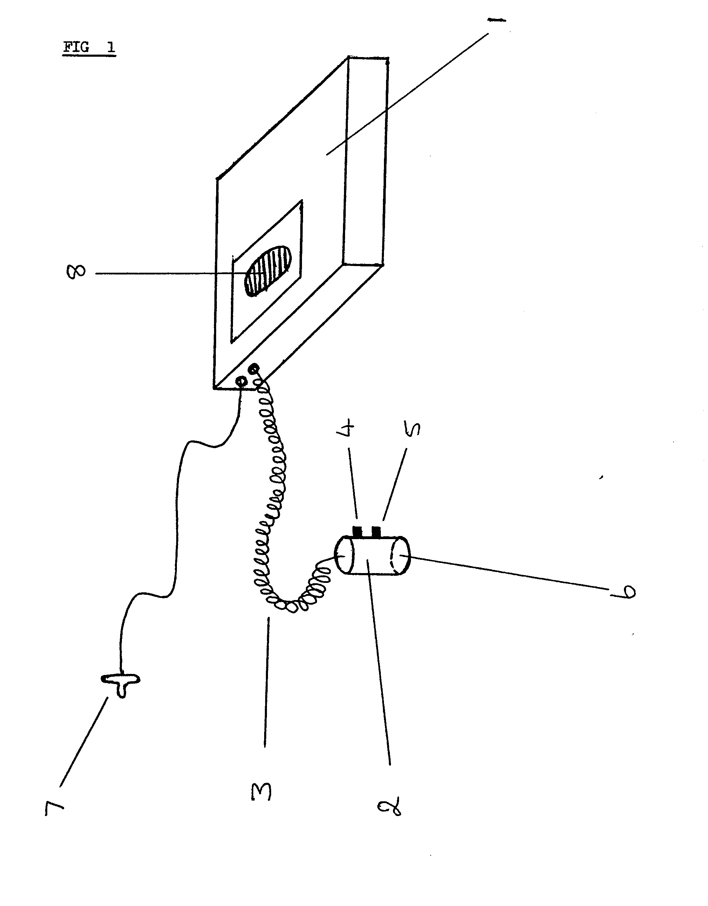

CLAIMS :

This invention has one claim. It is a portable electronic instrument to transmit informtaion to the blind person, by way of electronically generated speech. The information capable of being transmitted is that of all colours and various levels of illumination.

This invention provides the blind person with 2 sensor devices, a colour sensor and an illumination sensor.

The instrument is fully portable and battery powered. It will be simple to operate as required for blind operator use.

The blind persons world can be quite isolated but they need contact as much as any sighted person. This instrument can become a necessity for independence. This instrument will have two main categorical uses:-
1) For use by the totally blind ; or
2) For use by the colour blind.

Operation of the colour sensor is controlled by depressing the first button (4) on the sensor cylinder (2) and placing the sensor surface (6) on the object whose colour is required.

Operation of the illumination sensor is controlled by depressing the second button (5) on the sensor cylinder (2). The sensor surface (6) will sense the level of illumination in its immediate surroundings.

The colour or illumination sensed from either sensor, within the sensor cylinder (2), is then passed as analogue to the electronic hardware (1) via the attached cable (3). The electronic hardware (1) will then convert the analogue information to digital information which is then analysed.

After this analysis, the information originally sensed, for example, either a colour or a level of illumination, will be produced as electronically generated speech, that is, "RED" , "BLUE" , "LIGHT" or "DARK" etc, through the ear-piece (7) or through the speaker (8) on the case of the instrument (1).

FIG 1

7

8

3

4

2

5

6

1

-1-

0235460

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | IEEE TRANSACTIONS ON INSTRUMENTATION AND MEASUREMENT, vol. IM-33, no. 2, June 1984, pages 122-126, New York, US; D.T. NGUYEN et al.: "A vocalized color recognition system for the blind" * entire document * | 1 | A 61 F 9/08 G 09 B 21/00 |
| Y | DE-U-7 922 356 (AID ELECTRONIC GMBH) * claim 1; figure 1 * | 1 | |
| A | BE-A- 897 382 (GENICOT) * claims 1, 10 * | 1 | |
| A | US-A-4 119 811 (MORICCA et al.) * claim 1; figures 2-4 * | 1 | TECHNICAL FIELDS SEARCHED (Int. Cl.4) |
| | | | A 61 F 9/00 |

The present search report has been drawn up for all claims

| Place of search | Date of completion of the search | Examiner |
|---|---|---|
| BERLIN | 21-05-1987 | KANAL P K |